Europäisches Patentamt

European Patent Office

Office européen des brevets

(11) Veröffentlichungsnummer: **0 367 917**

**A2**

(12) # EUROPÄISCHE PATENTANMELDUNG

(21) Anmeldenummer: **89114909.8**

(22) Anmeldetag: **11.08.89**

(51) Int. Cl.5: **A61H 9/00, A61H 39/04**

(30) Priorität: **17.09.88 DE 8811822 U**

(43) Veröffentlichungstag der Anmeldung:
**16.05.90 Patentblatt 90/20**

(84) Benannte Vertragsstaaten:
**AT FR GB IT NL**

(71) Anmelder: **Lendermann, Willi**
**Schwabacher Strasse 514**
**D-8510 Fürth(DE)**

(72) Erfinder: **Lendermann, Willi**
**Schwabacher Strasse 514**
**D-8510 Fürth(DE)**

(74) Vertreter: **Kessel, Egbert, Dipl.-Ing. et al**
**Patentanwälte Dipl.-Ing. E. Kessel Dipl.-Ing.**
**V. Böhme Karolinenstrasse 27**
**D-8500 Nürnberg 1(DE)**

(54) **Vorrichtung zur wahlweisen Durchführung von Wasserpunktur oder Hydro-Ionisation.**

(57) Die Erfindung betrifft eine Vorrichtung zur wahlweisen Durchführung von Wasserpunktur oder Hydro-Ionisation, die eine Wasserzu- und -ableitung aufweist, wobei der mit mindestens einer Düse versehene Austritt der Wasserzuleitung und der Eintritt der Wasserableitung in einem Block zusammengefaßt sind, an den ein Rohrstutzen anschließt, in dem Prallflächen für die Hydro-Ionisation angeordnet sind. Eine solche Vorrichtung soll ohne Umbau für beide Nutzungsarten (Wasserpunktur bzw. Wassermassage einerseits und Hydro-Ionisation andererseits) verwendbar und leicht handhabbar sein, wobei die Umgebung während dieser Handhabung nicht naß werden soll. Erfindungsgemäß wird das dadurch erreicht, daß der Austritt der Wasserzuleitung im Block zwei alternativ zuschaltbare Kanäle aufweist, von denen der eine für die Wasserpunktur und der andere für die Hydro-Ionisation bestimmt ist, und daß die Prallflächen von Innenwandbereichen des Rohrstutzens und/oder von Oberflächenbereichen des Blocks gebildet sind.

FIG. 2

## Vorrichtung zur wahlweisen Durchführung von Wasserpunktur oder Hydro-Ionisation

Die Erfindung betrifft eine Vorrichtung zur wahlweisen Durchführung von Wasserpunktur oder Hydro-Ionisation, die eine Wasserzu- und -ableitung aufweist, wobei der mit mindestens einer Düse versehene Austritt der Wasserzuleitung und der Eintritt der Wasserableitung in einem Block zusammengefaßt sind, an den ein Rohrstutzen anschließt, in dem Prallflächen für die Hydro-Ionisation angeordnet sind.

Bei einem bekannten Gerät dieser Gattung wird zunächst der als Griff dienende Rohrstutzen mit dem Block verschraubt. Je nach der gewünschten Nutzungsart werden dann weiter für die Wasserpunktur bzw. Wassermassage auf den Rohrstutzen Trichter mit meist kleiner Öffnungsweite aufgeschraubt, während für die Hydro-Ionisation nur Trichter mit sehr großer Öffnungsweite in Betracht kommen, denen jedoch noch ein besonderer Ionisator zugeordnet werden muß. Dieser Ionisator besteht aus einem Gebilde, das die Form des Mantels eines sehr flachen Kegels besitzt, an dem drei spreizbare Beine angebracht sind, denen Haltefunktion zukommt; in der Einbaustellung ist die Kegelspitze des Ionisators den ankommenden Wasserstrahlen zugekehrt, so daß diese auf die äußere Kegelmantelfläche auftreffen und dort zerstäuben, worauf die negativ geladenen Wasserionen im Trichter und über diesen hinaus aufsteigen, während die positiv geladenen Wasserionen in der Wasserableitung abfließen.

Diesem bekannten Gerät haften jedoch etliche Mängel an. So hat es sich als besonders ungünstig erwiesen, daß im Falle der Nutzungsänderung (Übergang von Wasserpunktur bzw. Wassermassage auf Hydro-Ionisation oder umgekehrt) ein Umbau des Geräts erforderlich ist; abgesehen von dem damit verbundenen Zeitaufwand führt die Notwendigkeit des Umbaus häufig zu einem Unterlassen der zweiten Nutzungsart. Durch den Umbau verschleißen jedoch vor allem die Gewinde sehr stark, was im Hinblick darauf, daß es sich in aller Regel um Kunststoffteile handelt, die alsbaldige Funktionsunfähigkeit des Geräts bewirkt. Außerdem bereitet die Aufbewahrung der verschiedenen losen Bauteile (mehrere Trichter mit unterschriedlichen Öffnungsweiten, Ionisator usw.) erfahrungsgemäß Schwierigkeiten. Schließlich bedingt die Kombination Ionisator-Trichter mit großer Öffnungsweite einen erheblichen Spritzradius, so daß die Umgebung des Anwendungsorts recht naß wird.

Der Erfindung liegt die Aufgabe zugrunde, dieses bekannte Gerät dahingehend zu verbessern, daß es ohne Umbau für beide Nutzungsarten (Wasserpunktur bzw. Wassermassage einerseits und Hydro-Ionisation andererseits) verwendbar und leicht handhabbar ist sowie ein Naßwerden der Umgebung des Anwendungsorts vermeidet.

Diese Aufgabe wird erfindungsgemäß dadurch gelöst, daß der Austritt der Wasserzuleitung im Block zwei alternativ zuschaltbare Kanäle aufweist, von denen der eine für die Wasserpunktur und der andere für die Hydro-Ionisation bestimmt ist, und daß die Prallflächen von Innenwandbereichen des Rohrstutzens und/oder von Oberflächenbereichen des Blocks gebildet sind.

Aufgrund dieser Ausgestaltung ist für den Übergang von der einen Nutzungsart auf die andere lediglich ein Schaltvorgang erforderlich, der die Wasserzufuhr zu dem jeweils in Betracht kommenden Kanal des Aus tritts der Wasserzuleitung steuert; eines Umbaus bedarf es nicht mehr, zumal auch die Prallflächen von Bauteilen der Vorrichtung selbst und nicht von besonderen Zusatz-Bauteilen zur Verfügung gestellt werden. Darüber hinaus schafft der Verzicht auf einen Trichter mit großer Öffnungsweite nicht nur die Möglichkeit, das Naßwerden der Umgebung zu vermeiden, sondern läßt die negativ geladenen Wasserionen konzentriert und gebündelt aus dem Rohrstutzen austreten.

Gemäß einem vorteilhaften Merkmal der Erfindung ist der Anschluß der Wasserzuleitung an die Kanäle als Steckkupplung ausgebildet, so daß der Übergang von der einen Nutzungsart auf die andere nur ein einfaches Umstecken der Wasserzuleitung erfordert. Selbstverständlich könnte jedoch auch eine Ventilsteuerung verwendet werden.

In weiterer Ausgestaltung der Erfindung besitzt der Block eine kreisscheibenförmige Basis, auf der ein konzentrisch angeordneter Zylinder kleineren Durchmessers aufgebaut ist. Ein derartiger Block läßt sich auf einfache Weise bsp. als Kunststoffteil herstellen und im Rohrstutzen befestigen.

Zweckmäßigerweise ist in dem seitlich des Zylinders vorstehenden Bereich der Basis des Blocks der Eintritt der Wasserableitung untergebracht. Auf diese Weise werden die positiv geladenen Wasserionen, die sich auf der äußeren Kreisringfläche der Basis sammeln können, von dort unmittelbar abgeführt.

Es hat sich als besonders vorteilhaft erwiesen, wenn der Kanal für die Hydro-Ionisation den Block axial durchdringt und in einer Kammer mündet, die im Abstand von der oberen Basisfläche des Zylinders ange ordnet sowie in ihrer Seitenwand und/oder ihrem Boden mit Düsen versehen ist, wobei gemäß einem weiteren Merkmal der Erfindung, dem wesentliche Bedeutung zukommt, die Kammer einen zylindrischen Querschnitt aufweist, dessen Durchmesser kleiner als derjenige des zum Block gehörigen Zylinders ist. Die zentrale Anordnung der Kammer im Rohrstutzen und im Abstand vom Zylinder des Blocks läßt Flächen des Rohrstutzens und des Zylinders in die unmittelbare Nähe der Kammer rücken, die bei entsprechender Anordnung und Ausrichtung der Düsen als Prallflächen benutzt werden können.

Für die Anordnung und Ausrichtung der Düsen hat es sich gemäß einem weiteren Merkmal der Erfindung als zweckmäßig erwiesen, wenn diese in der Seitenwand der Kammer von innen nach außen leicht ansteigend und im Boden der Kammer von innen nach außen leicht nach außen verlaufend angeordnet sind. Auf diese Weise wird ein Rückprall der negativ geladenen Wasserionen auf die Seitenwand bzw. den Boden der Kammer vermieden; vielmehr werden diese Wasserionen sofort in Richtung der Austrittsöffnung des Rohrstutzens befördert.

Während die vorstehend angegebenen Weiterbildungen des erfindungsgemäßen Lösungsgedankens sich durchweg mit der Nutzungsart Hydro-Ionisation befaßt haben, sollen nun diejenigen Weiterbildungen beschrieben werden, welche die Nutzungsart Wasserpunktur bzw. Wassermassage betreffen.

Da ist erfindungsgemäß zunächst vorgesehen, daß der obere Bereich des zum Block gehörigen Zylinders als den mittig angeordneten Kanal konzentrisch umgebender Ringraum ausgebildet ist, in welchem der exzentrisch verlaufende Kanal für die Wasserpunktur mündet. Diese konstruktiv recht elegante Lösung ermöglicht vor allem eine sehr gedrängte, raumsparende Bauweise des Blocks.

In weiterer besonders vorteilhafter Ausgestaltung der Erfindung ist die obere Abdeckwand des Ringraums in ihrem von der Kammer nicht überdeckten Bereich mit Düsen versehen, die sich vorzugsweise parallel zur Längsachse des Blocks erstrecken. Aufgrund dieser Ausgestaltung können sich die aus den Düsen des Ringraums austretenden Wasserstrahlen ungehindert seitlich an der Kammer des Kanals für die Hydro-Ionisation vorbei parallel zur Längsachse des Rohrstutzens bewegen bzw. fortpflanzen.

Bei der weiter oben beschriebenen Anordnung und Ausrichtung der Düsen bilden gemäß einem weiteren Merkmal der Erfindung der der Seitenwand der Kammer radial gegenüberliegende Innenwandbereich des Rohrstutzens und/oder die Außenoberfläche der Abdeckwand des Ringraums die Prallflächen.

In der Zeichnung ist ein bevorzugtes Ausführungsbeispiel der Erfindung dargestellt.

Es zeigen

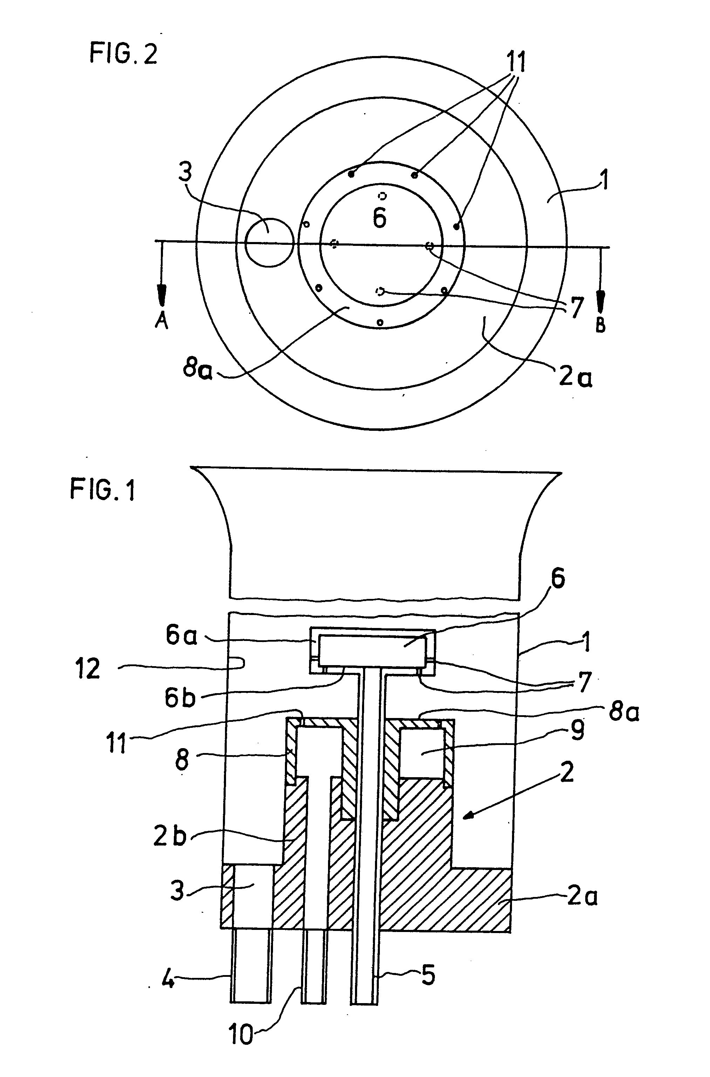

Fig. 1 einen Schnitt nach Linie A - B in Fig. 2 durch eine erfindungsgemäße Vorrichtung mit teilweise weggebrochenem Rohrstutzen und

Fig. 2 eine Draufsicht auf die in Fig. 1 dargestellte erfindungsgemäße Vorrichtung.

Die Vorrichtung besteht im wesentlichen aus einem an einem Ende trompetenartig erweiterten Rohrstutzen 1 und einem in dessen anderem Ende gehaltenen Block 2, der in eine Basis 2a und einen darauf konzentrisch angeordneten Zylinder 2b gegliedert ist. Bei dem gezeigten Ausführungsbeispiel sind die Basis 2a und der Zylinder 2b einstückig, z.B. als Kunststoffteil, ausgebildet.

In dem seitlich des Zylinders 2b vorstehenden Bereich der Basis 2a des Blocks 2 ist ein Eintritt 3 einer Wasserableitung 4 vorgesehen. Axial ist der Block 2 von einem Kanal 5 durchdrungen, der in einer zylindrischen Kammer 6 mündet, deren Seitenwand 6a und Boden 6b mehrere Düsen 7 aufweisen. Der Durchmesser der Kammer 6 ist kleiner als der Durchmesser des damit konzentrischen Zylinders 2b.

Auf den Zylinder 2b ist eine Kappe 8 aufgesetzt, die den gleichen Durchmesser wie der Zylinder 2b aufweist und den Kanal 5 unter Bildung eines Ringraums 9 konzentrisch umgibt. In den Ringraum 9 mündet ein Kanal 10, der den Block 2 exzentrisch parallel zu dessen Längsachse bzw. zum Kanal 5 durchdringt. In dem von der Kammer 6 nicht überdeckten Bereich der Abdeckwand 8a der Kappe 8 sind mehrere Düsen 11 vorgesehen.

Während die Düsen 11 das über den Kanal 10 zugeführte Wasser feinstrahlig seitlich an der Kammer 6 vorbei in den freien Raum des Rohrstutzens 1 vorantreiben, prallt das über den Kanal 5 zugeführte, aus den Düsen 7 der Kammer 6 austretende Wasser gegen den mit 12 bezeichneten Innenwandbereich des Rohrstutzens 1 bzw. gegen die Außenoberfläche der Abdeckwand 8a des Ringraums 9 und wird dort fein zerstäubt, wobei der negativ geladene Wasserstaub im Rohrstutzen 1 aufsteigt und der positiv geladene Wasserstaub auf die Basis 2a des Blocks 2 absinkt und durch den Eintritt 3 in die Wasserableitung 4 gelangt.

Die Kanäle 5 und 10 sind mittels einer - nicht dargestellten - Steckkupplung an die - ebenfalls nicht dargestellte - Wasserzuleitung anschließbar.

Bezugszeichenliste

1 Rohrstutzen
2 Block
2a Basis
2b Zylinder
3 Eintritt
4 Wasserableitung
5 Kanal
6 Kammer
6a Seitenwand
6b Boden
7 Düsen

8 Kappe
8a Abdeckwand
9 Ringraum
10 Kanal
11 Düsen
12 Innenwandbereich

## Ansprüche

1. Vorrichtung zur wahlweisen Durchführung von Wasserpunktur oder Hydro-Ionisation, die eine Wasserzu- und -ableitung aufweist, wobei der mit mindestens einer Düse versehene Austritt der Wasserzuleitung und der Eintritt der Wasserableitung in einem Block zusammengefaßt sind, an den ein Rohrstutzen anschließt, in dem Prallflächen für die Hydro-Ionisation angeordnet sind, **dadurch gekennzeichnet,** daß der Austritt der Wasserzuleitung im Block (2) zwei alternativ zuschaltbare Kanäle (5 und 10) aufweist, von denen der eine (10) für die Wasserpunktur und der andere (5) für die Hydro-Ionisation bestimmt ist, und daß die Prallflächen von Innenwandbereichen (12) des Rohrstutzens (1) und/oder von Oberflächenbereichen (8a) des Blocks (2) gebildet sind.

2. Vorrichtung nach Anspruch 1, **dadurch gekennzeichnet,** daß der Anschluß der Wasserzuleitung an die Kanäle (5 und 10) als Steckkupplung ausgebildet ist.

3. Vorrichtung nach den Ansprüchen 1 und 2, **dadurch gekennzeichnet,** daß der Block (2) eine kreisscheibenförmige Basis (2a) besitzt, auf der ein konzentrisch angeordneter Zylinder (2b) kleineren Durchmessers aufgebaut ist.

4. Vorrichtung nach Anspruch 3, **dadurch gekennzeichnet,** daß in dem seitlich des Zylinders (2b) vorstehenden Bereich der Basis (2a) des Blocks (2) der Eintritt (3) der Wasserableitung (4) untergebracht ist.

5. Vorrichtung nach den Ansprüchen 3 und 4, **dadurch gekennzeichnet,** daß der Kanal (5) für die Hydro-Ionisation den Block (2) axial durchdringt und in einer Kammer (6) mündet, die im Abstand von der oberen Basisfläche des Zylinders (2b) angeordnet sowie in ihrer Seitenwand (6a) und/oder ihrem Boden (6b) mit Düsen (7) versehen ist.

6. Vorrichtung nach Anspruch 5, **dadurch gekennzeichnet,** daß die Kammer (6) einen zylindrischen Querschnitt aufweist, dessen Durchmesser kleiner als derjenige des zum Block (2) gehörigen Zylinders (2b) ist.

7. Vorrichtung nach den Ansprüchen 5 und 6, **dadurch gekennzeichnet,** daß die Düsen (7) in der Seitenwand (6a) der Kammer (6) von innen nach außen leicht ansteigend und im Boden (6b) der Kammer (6) von innen nach außen leicht nach außen verlaufend angeordnet sind.

8. Vorrichtung nach Anspruch 3 und mindestens einem der folgenden, **dadurch gekennzeichnet,** daß der obere Bereich des zum Block (2) gehörigen Zylinders (2b) als den mittig angeordneten Kanal (5) konzentrisch umgebender Ringraum (9) ausgebildet ist, in welchem der exzentrisch verlaufende Kanal (10) für die Wasserpunktur mündet.

9. Vorrichtung nach Anspruch 8, **dadurch gekennzeichnet,** daß die obere Abdeckwand (8a) des Ringraums (9) in ihrem von der Kammer (6) nicht überdeckten Bereich mit Düsen (11) versehen ist.

10. Vorrichtung nach Anspruch 9, **dadurch gekennzeichnet,** daß die Düsen (11) sich etwa parallel zur Längsachse des Blocks (2) erstrecken.

11. Vorrichtung nach Anspruch 1 und mindestens einem der folgenden, **dadurch gekennzeichnet,** daß der der Seitenwand (6a) der Kammer (6) radial gegenüberliegende Innenwandbereich (12) des Rohrstutzens (1) und/oder die Außenoberfläche der Abdeckwand (8a) des Ringraums (9) die Prallflächen bilden.

EP 0 367 917 A2

FIG. 2

11

3

1

6

7

A

B

8a

2a

FIG. 1

6

6a

1

12

7

6b

8a

11

9

8

2

2b

3

2a

4

5

10